# EUROPEAN PATENT APPLICATION

(11) **EP 2 071 313 A1**
(43) Date of publication of application: **17.06.2009**
(21) Application number: 07828878.4
(22) Date of filing: 21.09.2007
(51) Int. Cl.: G01N 1/28, G01N 1/30, G01N 27/62, G01N 33/48

(54) **METHOD FOR DEPARAFFINIZATION OF PARAFFIN-EMBEDDED SPECIMEN AND METHOD FOR ANALYSIS OF PARAFFIN-EMBEDDED SPECIMEN**

(30) Priority: 28.09.2006 JP 2006265224
(71) Applicant: Shimadzu Corporation, Kyoto-shi Kyoto 604-8511 (JP)
(72) Inventor: SATO, Taka-aki, Kyoto-shi Kyoto 604-8511 (JP); AOKI, Yutaka, Kyoto-shi Kyoto 604-8511 (JP); SHIMADA, Takashi, Kyoto-shi Kyoto 604-8511 (JP); TOYAMA, Atsuhiko, Kyoto-shi Kyoto 604-8511 (JP)
(74) Representative: Wilhelms · Kilian & Partner Patentanwälte
(86) International application number: PCT/JP2007/069137
(87) International publication number: WO 2008/038813

(57) **Abstract**

The present invention provides a method for deparaffinization useful for mass spectrometric imaging from a paraffin-embedded specimen. A method for deparaffinization of paraffin-embedded specimen comprising the steps of: exposing a biological sample by subjecting a specimen in which the biological sample is embedded in paraffin, to an organic solvent that is compatible with the paraffin under heating condition, to make the paraffin be melted and dissolved in the organic solvent; and removing the paraffin from the specimen by separating the organic solvent dissolving the paraffin, from the exposed biological sample.

## Description

### TECHNICAL FIELD

The present invention pertains to medical and biological fields such as cell biology, pathology and biochemistry. The present invention relates to a pretreatment method for a paraffin-embedded specimen, and more specifically to a method for deparaffinization of a paraffin-embedded specimen. Also the present invention relates to a method for analysis of paraffin-embedded specimen, and particularly to a mass spectrometric imaging method of a paraffin-embedded specimen.

### BACKGROUND ART

### <Paraffin-embedded specimen>

Today, a great number of paraffin-embedded biological tissues used in pathological diagnoses and the like are preserved. The ability of acquiring information of biological molecules from these will allow investigation of diagnosis, therapy, pathologic finding, prognosis and the like in a retrospective manner using past cases, and hence is recognized as being a great advantage in a research of pathology of outbreak of disease, development of a new drug and so on.

In an analysis of a paraffin-embedded specimen, a pretreatment for removing paraffin from the specimen is conducted (deparaffinization). As a method of deparaffinization, typically, a treatment of dissolving and removing paraffin typically with xylene under a normal temperature environment is conducted. The treatment of dissolving and removing paraffin with xylene is typically repeated plural times (for example, about twice to 4 times). As another method of deparaffinization, Japanese Patent Application Laid-Open Publication No. 2005-221511 discloses a method of melting and removing an embedding medium by using a deparaffinization and a cell conditioning reagent which is inmiscible to paraffin without using an organic solvent.

### <Bioimaging>

In recent years, bioimaging techniques that directly observe cells and biological tissues for visually examining biological phenomena occurring in a living body have been developed and advanced. The bioimaging technique makes it possible to detect a biological molecule while keeping the positional information in the living body. In the bioimaging technique, a thin section of a biological tissue specimen (for example, a frozen section or a paraffin-embedded section) is used as a sample.

As a means in the bioimaging, microscopy is used in most cases.

As an example of imaging by the microscopy, for example, when a biological molecule such as a protein molecule is observed, an immunohistochemical technique is generally used. In this technique, imaging may be achieved, for example, by labeling a target biological molecule contained in a tissue section via a specific antibody, and detecting coloring, luminescence, fluorescence or the like of the label.

In the case of an immunohistochemical technique, a frozen section tends to be used as a tissue section from the viewpoint of keeping of antigenicity, and a paraffin-embedded section tends to be used from the viewpoint of keeping a form of a target tissue. When a paraffin-embedded section is used, a deparaffinization treatment and an antigen activation treatment are carried out. Concretely, as a deparaffinization treatment, as described above, a treatment of dissolving and removing paraffin typically with xylene in a normal temperature environment is conducted as described above.

Also as other means in bioimaging, mass spectrometry may be used (namely, mass spectrometric imaging).

As an example of mass spectrometric imaging, for example, after subjecting a protein molecule contained in a tissue section to a treatment such as digestion as needed, mass spectrometry is conducted for a plurality of positions on a surface of the tissue section, and an image is formed from mass spectrums obtained for respective positions on a surface of the tissue section.

In the mass spectrometric imaging, a frozen section of biological tissue is typically used as a sample.

For example, Stoeckli, M.; Chaurand, P.; Hallahan, E. D., Caprioli, M. R. Nature Med. 7, 493-496 (2001), Chaurand, P.; Schwartz. A. S; Caprioli, M. R. Anal. Chem., 76, 86A-93A (2004), and Chaurand, P.;Schwartz, A.S.; Billheimer, D.; Xu, J.B; Crecelius, A.; Caprioli, M. R. Anal. Chem., 76, 1145-1155 (2004) and so on report to subject a frozen biological tissue section to a MALDI mass Spectrometry, thereby obtaining MS spectrum.

Also Japanese Patent Application Laid-Open Publication No. 2004-347594 reports subjecting a frozen biological tissue section to the MALDI mass spectrometry, and obtaining a MS spectrum.

Very recently, there is also reported to execute mass spectromefric imaging by using a paraffin section of a biological tissue. Concretely, 54th ASMS Conference on Mass Spectrometry collected programs, pl47 (Abstract of Session: Imaging MS II Code: ThP18 Time Slot/Poster Number: 333) reports that a paraffin section is subjected to a treatment by a usual immunocytochemical technique, and MALI mass spectrometric imaging is conducted directly by using reactive matrix.

On the other hand, although not being the example that mass spectrometry is conducted directly on a biological tissue, BIO VIEW (TAKARA BIO INC.), 03/12, No.44, P13-16 reports that a specific cell is collected and processed by laser capture micro-dissection or the like from a paraffin section, and a protein extracted from the collected cell is treated with an enzyme and subjected to LC/MS analysis.
Patent Document 1: Japanese Patent Application Laid-Open Publication No. 2005-221511
Patent Document 2: Japanese Patent Application Laid-Open Publication No. 2004-347594
Non-Patent Document 1: Stoeckli, M.; Chaurand, P.; Hallahan, E. D., Caprioli, M. R. Nature Med. 7, 493-496 (2001)
Non-Patent Document 2: Chaurand, P.; Schwartz. A. S; Caprioli, M. R. Anal. Chem., 76, 86A-93A (2004)
Non-Patent Document 3: Chaurand, P.;Schwartz,A.S.; Billheimer, D.; Xu, J.B; Crecelius, A.; Caprioli, M. R. Canal. Chem., 76, 1145-1155 (2004)
Non-Patent Document 4: 54th ASMS Conference on Mass Spectrometry collected programs, p147
Non-Patent Document 5: BIO VIEW (TAKARA BIO INC.), Dec., 2003, No.44, P13-16

### DISCLOSURE OF THE INVENTION

### Object of the Invention

A bioimaging technique using a paraffin-embedded section as a sample is superior to the case where a frozen section is used as a sample in that it is also effective in pathological diagnosis or the like which allows retrospective study and has versatility.

Since the bioimaging technique using mass spectrometry identifies a biological molecule itself, it is possible to directly grasp a biological molecule based on the quantification result. On the other hand, the bioimaging technique using microscopy grasps a biological molecule indirectly. Therefore, the mass spectrometric imaging is superior in the point that accuracy of an analytical result is greater.

Therefore, ability of conducting mass spectrometric imaging using a paraffin-embedded section will be a very useful technique.

Abstract in p.147, collected programs for 54th ASS Conference on Mass Spectrometry, p147 reports conducting a mass spectrometric imaging by using a paraffin-embedded section. Since this abstract describes subjecting a paraffin section to a treatment based on a normal immunohistochemical technique, it is naturally expected that concretely a deparaffinization treatment is conducted typically using xylene under a normal temperature condition.

Although such a conventional deparaffinization treatment method has been approved in common-sense manner because it satisfies an immunohistochemically significant analytical result, it may not be said that the method is approved similarly in a mass spectrometric approach. As one reason, when mass spectrometry is used, the influence of paraffin remaining in a biological sample on an analytical result is considered to be larger than influence in microscopy. That its, the remaining paraffin will cause a mass spectrum of a small amount of information regarding a target biological molecule, and will exert an adverse affect on quality of the analysis.

Therefore, in the method described in the above abstract, quality of imaging is low because a mass spectrum of a sufficient information amount is not obtained.

In consideration of this, the inventors of the present application notified that stricter requirements should be imposed on the condition (for example, degree of exposure) of a biological sample after removal of paraffin in order to obtain a mass spectrum which excels in information amount and to conduct a mass spectrometric imaging of high quality.

Accordingly, it is an object of the present invention to provide a method for deparaffinization useful for mass spectrometric imaging from a paraffin-embedded specimen. It is also an object of the present invention to provide a method capable of conducting a mass spectrometric imaging of high quality from a paraffin-embedded specimen.

### Summary of the Invention

The present invention includes the following (1) to (14). The following (1) to (7) are directed to a method for deparaffinization of a paraffin-embedded specimen, and the following (8) to (14) are directed to a method for an analysis of a paraffin-embedded specimen.

In the present invention, the term "paraffin" broadly implies embedding media for biological samples, for use any analyses including morphological, immunohistochemical, and enzymehistochemical analyses. That is, paraffin used in the present invention may be a petroleum-based paraffin wax alone, or mixtures in which any other ingredients are added to the petroleum-based paraffin wax ass base, for the purpose of improvement in quality of the embedding medium. Here, the petroleum-based paraffin wax refers to mixtures of hydrocarbons that are derived from petroleum and solid at a normal temperature.

### <Method for deparaffinization of a paraffin-embedded specimen>

(1) A method for deparaffinization of paraffin-embedded specimen comprising the steps of:
   exposing a biological sample by subjecting a specimen in which the biological sample is embedded in paraffin, to an organic solvent that is compatible with the paraffin under heating condition, to make the paraffin be melted and dissolved in the organic solvent; and
   removing the paraffin from the specimen by separating the organic solvent dissolving the paraffin, from the exposed biological sample.

   In the above (1), the expression "compatibles means that the substances (concretely, a liquid paraffin and an organic solvent) exhibit solubility of such an extent that phase separation will not occur. The term "exposing" of a biological sample means that a biological sample gets exposed as a result of elution of paraffin from the specimen in which the biological sample is embedded in the paraffin.
   The following (2) is directed to one aspect of the method for deparaffinization of the present invention viewed from a concrete operation.
(2) The method for deparaffinization of a paraffin-embedded specimen according to (1), wherein in the step of exposing the biological sample, the specimen in which the biological sample is embedded in the paraffin is immersed and retained in the organic solvent that is heated, and
   in the step of removing the paraffin, the specimen retained in the organic solvent is drawn up from the organic solvent.
(3) The method for deparaffinization of a paraffin-embedded specimen according to (1) or (2), wherein the heating condition is a temperature condition of 45 to 70°C.
(4) The method for deparaffinization of a paraffin-embedded specimen according to (1) or (2), wherein the organic solvent is selected from the group consisting of xylene, chloroform, diethyl ether, lemosol, and alcohols.
   In the above (4), the alcohols include methanol, isopropyl alcohol and so on.
(5) The method for deparaffinization of a paraffin-embedded specimen according to any one of (1) to (4), wherein the specimen is held on a surface of an electrically conductive support.
   The method according to the above (5) enables to be applied as a pretreatment method that is useful especially when mass spectrometry is conducted from a paraffin-embedded specimen.
(6) The method for deparaffinization of a paraffin-embedded specimen according to any one of (1) to (5), wherein the specimen is derived from a living body suffering from disease selected from the group consisting of cancer, Alzheimer disease, Parkinson disease, ischemic cerebrovascular disease, and ischemic cerebrovascular disease.
(7) The method for deparaffinization of a paraffin-embedded specimen according to any one of (1) to (6), wherein the specimen is a specimen for analyzing pharmacokinetics.

In the above (7), the expression "specimen for analyzing pharmacokinetics" means a specimen used for verifying and evaluating the potential as a pharmaceutical agent from the viewpoint of pharmacokinetics (absorption, distribution, metabolism, and excretion) and, concretely is a specimen derived from a living body administered with a drug.

In the method for deparaffinization of the present invention, the paraffin-embedded specimen may be one having been stored for a long term.

### <Analytical method of paraffin-embedded specimen>

(8) A method for analysis of a paraffin-embedded specimen comprising the steps of:
   removing a paraffin from a specimen in which a biological sample is embedded in the paraffin using the method according to any one of (1) to (7); and
   measuring the biological sample by a mass spectrometer.

   According to the present invention, since heating is conducted in deparaffinization according to any one of the above (1) to (7), the organic solvent having adhered to the biological sample after deparaffinization vaporizes by the heat. Therefore, as will be described in the (9) below, a digestion treatment can be conducted without conducting a hydration treatment that is conventionally conducted before the digestion treatment.
   The following (9) is directed to one aspect of a method for analysis of the present invention that further executes a digestion step.
(9) The method for analysis of a paraffin-embedded specimen according to (8), further comprising the step of subjecting the biological sample to a digestion treatment, after the step of removing paraffin and before the step of measuring by a mass spectrometer.
   By the digestion treatment described in the above (9), proteins in the biological sample are digested, and more mass spectrum peaks can be obtained.
   The following (10) and (11) are directed to one aspect of the method for analysis of the present invention that further execute a hydration step.
(10) The method for analysis of a paraffin-embedded specimen according to (8), further comprising the step of subjecting the biological sample to a hydration treatment, after the step of removing paraffin and before the step of measuring by a mass spectrometer.
   By the hydration treatment as described in the above (10), paraffin that may remain in a minute amount after deparaffinization is effectively washed off, so that more mass spectrum peaks can be obtained.
   The following (11) is directed to an aspect of further conducting an enzymatic treatment in one aspect of a method for analysis that conducts a hydration treatment.
(11) A method for analysis of a paraffin-embedded specimen according to (8), further comprising a step of subjecting the biological sample to a hydration treatment and a digestion treatment, after the step of removing paraffin and before the step of measuring by a mass spectrometer.
   The following (12) to (14) are directed to one aspect of the method for analysis of the present invention using a MALDI mass spectrometer.
(12) The method for analysis of a paraffin-embedded specimen according to any one of (8) to (11), wherein in the step of measuring by a mass spectrometer, a matrix-assisted laser desorption/ionization mass spectrometer is used as the mass spectrometer.
(13) The method for analysis of a paraffin-embedded specimen according to (12), wherein 2,5-dihydroxybenzoic acid is used as a matrix, a solution of 40 mg/mL to a saturated concentration of the matrix is added dropwise to the biological sample by using an inkjet mechanism, and measurement is conducted using the matrix-assisted laser desorption/ionization mass spectrometer.
   According to the method of the above (13), since it is possible to deposit microcrystals of the matrix uniformly, it is possible to effectively ionize the biological molecule to be measured.
(14) The method for analysis of a paraffin-embedded specimen according to (13), wherein the matrix solution is added dropwise at a pitch of 100 to 200 µm.
   According to the method of the above (14), since it is possible to deposit microcrystals of the matrix uniformly on the biological sample, it is possible to stably and effectively ionize the biological molecule to be measured on the biological sample, and to know its localization together with precise positional information. (Since it is no longer required to visually check whether uniform crystals generate, restriction on measuring conditions is no longer required).

According to the present invention, its possible to provide a method for deparaffinization which is useful for mass spectrometry from a paraffin-embedded specimen, in particular for mass spectrometric imaging.

Furthermore, according to the present invention, it is possible to provide a method capable of conducting mass spectrometry, from a paraffin-embedded specimen, in particular, mass spectrometric imaging with excellent quality. Furthermore, by obtaining data from a pathological specimen that has been stored for a long time from the past using the method of the present invention, it is possible to profile an expressed protein, and to correlate an expression pattern of protein with disease.

### BRIEF DESCRIPTION OF THE DRAWINGS

Fig. 1 is a graph showing a result of mass spectrometry from a paraffin section using a conventional method for deparaffinization (Comparative Example 1), and a result of mass spectrometry from a paraffin section using a method for deparaffinization (heating time: 10 min.) according to the present invention (Example 1) in the form of relation between the temperature at the time of deparaffinization and the number of peaks in mass spectrum;
Fig. 2 is a graph showing a result of mass spectrometry from a paraffin section using a method for deparaffinization (heating temperature: 60°C) according to the present invention (Example 2) in the form of relation between the heating time at the time of deparaffinization and the number of peaks in mass spectrum;
Fig. 3 is a graph showing a result of mass spectrometry from a paraffin section using a method for deparaffinization (heating condition: 60°C-10 min.) according to the present invention (Example 3), in the form of relation between the dispensing pitch of a matrix solution and the number of peaks in mass spectrum;
Fig. 4 is a graph showing a result of mass spectrometry from a paraffin section according to a method for analysis of the present invention (deparaffinization conditions: 60°C-10 min.) in which a hydration treatment is conducted (Example 3) and a result of mass spectrometry from a paraffin section according to a method for analysis of the present invention (deparaffinization condition: 60°C-10 min.) in which a hydration treatment is not conducted (Example 4), in the form of relation between the dispensing pitch of matrix solution and the number of peaks in mass spectrum;
Fig. 5(a) is a mass spectrum obtained as a result of mass spectrometry from a paraffin section according to a method for analysis of the present invention (Example 3: deparaffinization condition is 60°C, 10 min.); and Fig. 5(b) is a mass spectrum obtained as a result of mass spectrometry from a frozen section according to a conventional method for an analysis (Comparative Example 2);
Fig. 6(a) is an actual image of a paraffin section analyzed in the present invention; and Fig. 6(b) is an actual image of a paraffin section after dispensing of a matrix;
Fig. 7 shows images (a) to (f) obtained by mass spectrometric imaging of the paraffin section according to a method for analysis of the present invention (deparaffinization condition: 60°C-10 min., hydration treatment: conducted, matrix solution dispensing pitch: 150 µm);
Fig. 8 shows images (a') to (f') obtained by superimposing an actual image (b) after dispensing of matrix in Fig. 6, on the images (a) to (f) of Fig. 7;
Fig. 9(a) is a photograph of a crystal generated when a matrix solution of high concentration (50 mg/mL) is added dropwise with a micro pipette; and Fig. 9(b) is a photograph of a crystal generated when a matrix solution of high concentration (50 mg/mL) is added dropwise with an inkjet mechanism;
Fig. 10(a) is a photograph of a crystal generated by a matrix solution of low concentration (10 mg/mL); and Fig. 10(b) is a photograph of a crystal generated by a matrix solution of high concentration (50 mg/mL); and
Fig. 11 is a schematic view showing the appearance that matrix is two-dimensionally dispensed on a biological sample.

### MODES FOR CARRYING OUT THE INVENTION

### [1. Paraffin-embedded specimen]

### [1-1. Paraffin]

As a paraffin-embedded specimen in the present invention, specimens that are to become research targets in any analyses including morphologic, immunohistochemical, and enzymehistochemical analyses, and typically specimens in the form of paraffin sections are intended. Paraffin in the present invention embraces those used as embedding media in such any analyses.

One example of a paraffin-embedded specimen in the present invention is a specimen prepared by using a petroleum-based paraffin wax alone as an embedding medium. Here, the petroleum-based wax refers to a mixture of hydrocarbons that are derived from petroleum and are solid at normal temperature. Further, the term hydrocarbons normally means saturated hydrocarbons having a molecular weight of about 300 to 500 which comprises linear hydrocarbons (normal paraffins) with an average carbon number of about 20 to 35 as a main component.

Another example of paraffin-embedded specimen according to the present invention is a specimen prepared by using an embedding medium containing the above-described petroleum-based paraffin wax as a base and further containing additional ingredients. As such additional ingredients, any ingredient that may be added for the purpose of improving quality of the embedding medium or the like are accepted. As an example of an embedding medium in which the additional ingredients are blended, an embedding medium in which a petroleum-based microcrystalline wax, polyisobutylene, an ethylene-vinyl acetate copolymer and polybutene are blended as the additional ingredients for the purpose of improving workability in slicing and cracking resistance at low temperature (for example, Japanese Patent Application Laid-Open Publication No. 2002-107354); an embedding medium in which polyisobutylene, an ethylene-vinyl acetate copolymer and saturated fatty acid are blended as the additional ingredients for the purpose of lowering the melting point, and improving workability in slicing and cracking resistance at low temperature (for example, Japanese Patent Application Laid-Open Publication No. 2004-212391) and the like are recited.

A melting point of the paraffin used in a paraffin-embedded specimen of the present invention is, for example, about 45 to 70°C (value measured in conformance with JIS K-2235), though it depends on its ingredients and composition.

### [1-2. Biological sample]

As described above, as a paraffin-embedded specimen in the present invention, specimens that are to become research targets in any analyses including morphologic, immunohistochemical, and enzymehistochemical analyses are intended. In many cases, it is a sliced specimen of a formalin-fixed paraffin-embedded (FFPE) biological sample. Therefore, such a specimen may be derived from any organisms. As for animals, amphibian, reptile, bird, mammalian and the like are widely accepted, and particularly preferred is a specimen derived from mammalian. Among these, a specimen derived from mouse or human being is more preferred. The specimen may be any of full-body specimen, organ specimen, tissue specimen, embryo specimen and cell specimen of such a living body. Further, when the specimen is a pathologic specimen, the disease from which the living body suffers may be any of cancer, Alzheimer disease, Parkinson disease, ischemic cerebrovascular disease, and ischemic heart disease.

The above-described specimen may be a specimen for an analysis of pharmacokinetics. A specimen for analysis of pharmacokinetics is a specimen for verifying and evaluating the potentiality as a pharmaceutical agent from the viewpoint of dynamics in the body (absorption, distribution, metabolism and excretion), and concretely a specimen derived from a living body administered with a drug. In the analysis of such a specimen, presence of a pharmaceutical drug having reached a target site is examined, for example, by detecting a biological molecule to which the pharmaceutical agent binds.

### [1-3. Support]

The above specimen may be held on a surface of a support such as a glass support, a resin support or a metallic support, or may be transferred electrically to a resin support, for example, to membrane. As the membrane, polyvinylidene difluoride (PVDF), nitrocellulose, polyamide, polyethylene and the like organic synthetic polymers and derivative thereof may be recited. As the polyamide, nylon and the like may be recited. These supports for holding and transferring a specimen may be determined appropriately by a person skilled in the art according to which type of analysis the specimen is subjected after a deparaffinization treatment by the method of the present invention.

When an analysis using mass spectrometry is conducted after a deparaffinization treatment, the one having electric conductivity may be used as a support. As such an electrically conductive support, for example, a sample plate for mass spectrometry, which is a metallic support is often used, however, a support coated with an electrically conductive substance may also be used without limited to the above. In this case, the material of the coated support is not particularly limited, and concretely those exemplified above. The electrically conductive substance is not particularly limited, and concrete examples include indium tin oxide (ITO) and the like. More concrete examples of a support coated with an electrically conductive substance include indium tin oxide-coated slide glass, and indium tin oxide-coated sheet.

A specimen may be used so as to be held on a surface of such a electrically conductive support by stricking, or so as to be transferred to a resin support, e.g. a membrane, and then held on an electrically conductive support by bonded or the like. For holding, fixation using an electrically conductive double-sided adhesive tape may be conducted. When the electrically conductive support itself is in the form of a sheet, the electrically conductive sheet on which the specimen is held may further be used while it is stuck on a support such as a plate.

In the present invention, it is particularly preferred that a specimen is held on a surface of an electrically conductive support.

### [2. Deparaffinization treatment]

### [2-1. Deparaffinization Operation]

The method for deparaffinization of the present invention comprises the steps of: exposing a biological sample by subjecting a specimen in which the biological sample is embedded in paraffin, to an organic solvent that is compatible with the paraffin under heating condition, to make the paraffin be melted and dissolved in the organic solvent; and removing the paraffin from the specimen by separating the organic solvent dissolving the paraffin, from the exposed biological sample. The above two steps may be conducted stepwise, or at once. Concrete examples of operation will be described below.

For example, such an operation may be carried out that in the step of exposing a biological sample, the specimen in which the biological sample is embedded in paraffin is immersed and retained in the organic solvent which is heated, and then in the step of removing paraffin, the specimen retained in the organic solvent is drawn up from the organic solvent.

In this example, before immersing the paraffin-embedded specimen in the heated organic solvent, the paraffin-embedded specimen may be heated in advance to melt the paraffin. Further, the heated organic solvent may be prepared by heating the organic solvent using a warm water bath.

Also such an operation may be conducted that, in the step of exposing the biological sample, the specimen in which the biological sample is embedded in paraffin is immersed in the organic solvent, and temperature of the organic solvent is raised and retained at an appropriate temperature; and then in the step of removing paraffin, the specimen retained in the organic solvent is drawn up from the organic solvent.

In the present invention, since elution of paraffin is effectively conducted, it is typically sufficient to conduct the operation comprising immersion into the organic solvent and drawing up only once. The operation may be conducted plural times in consideration of the heating temperature.

Furthermore, for example, the step of exposing the biological sample and the step of removing paraffin may be conducted at once by continuously applying a heated organic solvent onto the specimen in which the biological sample is embedded in paraffin.

In this example, before applying the heated organic solvent on the paraffin-embedded specimen, the paraffin-embedded specimen may be heated in advance to melt paraffin. The heated organic solvent may be prepared by heating the organic solvent by using a warm water bath.

Furthermore, after conducting the operation as exemplified above, further washing may be conducted by immersing and retaining in a warmed organic solvent (with no dissolved paraffin) or applying the warmed organic solvent, whereby the step of removing the paraffin may be conducted more strictly.

### [2-2. Heating condition

In the present invention, a heating condition or a temperature control condition is defined as a temperature condition around the melting point of paraffin. By applying such a temperature setting, it is possible to cause occurrence of a melting phenomenon of paraffin itself, so that it is possible to make the melted paraffin be dissolved (compatible) in the organic solvent. It is considered that this may make it possible to remove the paraffin having permeated in the biological sample more efficiently compared to the conventional method wherein solid paraffin is dissolved in an organic solvent. That is, the biological sample embedded in the paraffin is exposed more preferably compared to conventional cases. Therefor, the influence of remaining paraffin is smaller than those in conventional cases in later analysis of biological sample. This appears as a particularly good result when mass spectrometry is used in an analysis of a subsequent analysis of a biological sample.

A concrete heating temperature is not particularly limited because a melting point differs depending on the kinds of paraffin, and may be determined appropriately by a person skilled in the art. For example, as described above, since melting point of paraffin is, for example, about 45 to 70°C, the heating condition may also be 45 to 70°C. There is also the case that 55 to 65°C is preferred. Examples demonstrated that a mass spectrum of abundant information amount can be obtained when the deparaffinization treatment according to the present invention is conducted at a heating condition of 55 to 65°C, and then the specimen having subjected to the deparaffinization treatment is subjected to mass spectrometry. Below the above range, efficient melting of paraffin tends not to occur, and sufficient deparaffinization tends not to occur, so that a mass spectrum of a poor information amount tends to be obtained, whereas over the above range, a damage of a biological sample itself tends to be more likely to occur.

As for the heating time, there is no particular limitation, and determination maybe made by a person skilled in the art appropriately in consideration of a heating temperature, an embedding substance and the like various factors. For example, it may be about 5 to 20 minutes, and more preferably about 10 to 15 minutes. There are some cases that, under a temperature condition of about 60°C, about 10 to 15 minutes may be particularly preferred. Below the above range, sufficient deparaffinization tends not to occur, and a mass spectrum of a poor information amount tends to be obtained, whereas over the above range, a damage of a biological sample itself tends to be more likely to occur.

### [2-3. Organic solvent]

As the organic solvent, any organic solvents that are compatible with paraffin, more concretely, any organic solvents may be used without any limitation insofar as they exhibit solubility of such an extent that will not cause phase separation with liquid paraffin (namely, melted paraffin). For example, an organic solvent which is used as an intermediate agent for replacing for a dehydrating agent in a biological sample may be used after replacing water in the biological sample with a dehydrating agent, and before permeation of paraffin in the biological sample, in an embedding operation with paraffin. An example of organic solvent in the present invention may be selected from xylene, chloroform, diethyl ether, lemosol, and alcohols (for example, methanol, isopropyl alcohol and the like). These organic solvents may be used solely or in mixture of two or more kinds.

### [3. Biological sample having subjected to deparaffinization treatment]

Since paraffin is effectively removed from the paraffin-embedded specimen according to the method for deparaffinization of the present invention, the resultant biological sample is well exposed. There is a possibility that a heating condition in the deparaffinization treatment in the present invention provides the biological sample obtained by the treatment with not only a physically preferable effect such as exposure of a biological sample but also other, for example, a biochemically preferable effect.

Furthermore, the used organic solvent vaporizes effectively by the heating condition in the deparaffinization treatment of the present invention. Therefore, unlike a biological sample obtained by a conventional method for deparaffinization, in a biological specimen obtained by the method for deparaffinization of the present invention, an organic solvent is effectively removed. Therefore, in the case that treatments such as a dying treatment and a digestion treatment using an aqueous reagent is conducted, prior to such treatments a hydration treatment that is typically conducted in conventional arts is not necessarily required. Thus, the heating condition in the deparaffinization treatment of the present invention provides a preferred effect from the viewpoint of workability of the biological sample obtained by the treatment.

A biological sample obtained through the deparaffinization of the present invention may be subjected to any type of analysis. For example, DNA analysis, mRNA analysis, protein analysis and the like may be recited. As methods of these analyses, approaches may be made from any points of view including morphological, immunohistochemical, and enzymehistochemical approaches.

When a biological sample that is obtained while conducting the method for deparaffinization of the present invention is subjected to analysis, a pretreatment for the analysis is further conducted appropriately as needed by a person skilled in the art. As such a process, for example, a digestion treatment and a hydration treatment are recited.

In particular, when a biological molecule such as protein is analyzed by using mass spectrometry, the method for deparaffinization of the present invention is useful as a pretreatment method. The present invention also provides a method for analysis in such a case. That is, the present invention provides a method for analysis of a paraffin-embedded specimen comprising the steps of: removing the paraffin from a specimen in which a biological sample is embedded in paraffin using the above method; and measuring the biological sample by a mass spectrometer.

In the method for an analysis of the present invention, a hydration step and/or a digestion step may further be conducted between the deparaffinization step and the mass spectrometry step. When both of a hydration step and a digestion step are conducted, the digestion step is conducted after the hydration step.

### [4. Digestion treatment]

When a digestion treatment is executed, concrete operation thereof and various conditions may be determined appropriately by a person skilled in the art. For example, a solution of protease such as trypsin may be added and incubated in a wet condition.

By conducting such a digestion treatment, it is possible to obtain a mass spectrum containing more peaks derived from target biological molecules when a biological sample is subjected to mass spectrometry. Concretely, a mass spectrum, having at least the information amount that is obtainable when mass spectrometry is conducted on a frozen section having subjected to a digestion treatment, is ensured.

In the digestion step, as a method of supplying a regent with a biological sample, dispensing (namely, supplying liquid drop of minute amount) may be conducted. The dispensing operation makes it possible to supply a microscopic region of the biological sample with the reagent. For conducting the dispensing, any device capable of supplying a minute amount of the reagent solution may be used without any particular limitation. In particular, a dispenser equipped with an inkjet mechanism is preferably used. As a concrete inkjet mechanism, a mechanism using a piezoelectric element or the like is recited. As such a dispensing device, a chemical printer CHIP-1000 (made by SHIMADZU CORPORATION) or the like is recited.

### [5. Hydration treatment]

When a hydration treatment is executed, concrete operation thereof and various conditions may be determined appropriately by a person skilled in the art. Typically, an organic solvent that is compatible with both the organic solvent used in the above deparaffinization (namely, the organic solvent that is compatible with paraffin) and water is used. As such an organic solvent for the hydration treatment, the alcohol is used in most cases. Concretely, by using an organic solvent for the hydration treatment, followed by aqueous solutions in which the organic solvent is serially diluted, a biological sample may be hydrated.

By conducting such a hydration treatment, the organic solvent for deparaffinization that remains unvaporized to remain in the biological sample after the deparaffinization is effectively washed off. Therefore, when a biological sample is subjected to mass spectrometry, more peaks originating from a target biological molecule can be obtained.

### [6. Mass spectrometry]

In the method for an analysis of the present invention, a biological sample obtained by conducting the above deparaffinization treatment, or a biological sample having subjected to the additional digestion treatment and/or hydration treatment described above as appropriate is subjected to a mass spectrometry step. As a mass spectrometer used in this step, a matrix-assisted laser desorption/ionization (MALDI) mass spectrometer is preferred. For example, as an apparatus that is preferred from the point of capability of second or higher order of multi-stage MS analysis, AXIMA-QIT (made by SHIMADZU CORPORATION) is exemplified. By using a mass spectrometer capable of second or higher order of multi-stage MS analysis, it is possible to identify a measured molecule.

When a MALDI mass spectrometer is used, there is no limitation for a matrix. For the analysis of protein, for example, 2,5-dihydroxybenzoic acid (DHB), α-cyano-4-hydroxycinnamic acid (α-CHCA), 3,5-dimethoxy-4-hydroxycinnamic acid (sinapinic acid) and the like may be used. Such a matrix is used in the form of a matrix solution by dissolving it in an appropriate solvent. The solvent and its composition for dissolving the matrix may be appropriately determined by a person skilled in the art, and an aqueous solution of acetonitrile (ACN) - trifluoroacetic acid (TFA) is often used. Composition of the aqueous solution of acetonitrile (ACN) - trifluoroacetic acid (TFA) may be determined appropriately by a person skilled in the art. For example, it may be an aqueous solution containing 25 to 50 (v/v)% ACN - 0.05 to 1 (v/v)% TFA.

### [6-1. Microdispensing of high concentration DHB]

However, when DHB is used as a matrix, it is particularly preferred to use it at higher concentration than that widely used by a person skilled in the art heretofore. For example, it may be 40 mg/mL to saturated concentration, and more preferably about 50 mg/mL. Below this range, uniformly deposited crystals tend to be difficult to generate. The above saturated concentration is a saturated concentration at the temperature in the operation environment. The operation environment is a temperature which is generally so-called room temperature, concretely 15 to 30°C, and preferably 20 to 25°C.

In addition, when a DHB solution of such high concentration is used, it is preferred to supply the biological sample with a matrix using a dispenser equipped with an inkjet mechanism. As to the dispenser equipped with an inkjet mechanism, a mechanism utilizing a piezoelectric element or the like is recited as is described in the above item "4. Digestion treatment", and such a dispenser, a chemical printer CHIP-1000 (made by SHIMADZU CORPORATION) or the like may be recited.

A dispenser equipped with an inkjet mechanism is able to dispense liquid droplets of a picoliter order to a microscopic region. Concretely, an amount of a reagent that is dispensed at one discharge may be controlled to, for example, about 100 pL, and however, it may be an even smaller amount depending on the mechanism of the inkjet. For example, discharge of about 100 pL produces a minimum dispensing range of about 100 µm in a diameter.

When DHB is prepared in a high concentration, and dispensed into a microscopic region by an inkjet technique, uniformly deposited crystals generate. Crystals may be produced by overlaying the DHB solution in the same site. When dispensing is conducted in such an overlaying manner, overlaying may be conducted about 5 to 80 times, preferably 15 to 40 times, for each site.

Crystals in the microscopic region will generate both for the case of a biological sample obtained by deparaffinization of a paraffin section as is the case of the present invention, and for the case of a frozen section. However, a biological sample from a paraffin section tends to form crystals more easily in a microscopic region compared to a frozen section because liquid droplet are less likely to extend.

In MALDI mass spectrometry, uniformity of crystals is a factor that is greatly involved in quality of an analysis (for example, quantitative performance and reproducibility). Therefore, dispensing a high concentration DHB solution by an inkjet technique allows production of a crystal in which a matrix is uniformly deposited, which is preferable for effective ionization of a molecule to be measured. Therefore, it is preferable in that a mass spectrum having excellent quantitative performance and reproducibility can be obtained. Furthermore, this point is particularly important in an automatic analysis. Additionally, unlike α-cyano-hydroxycinnamic acid and the like, DHB is less likely to get clogged in an inkjet nozzle even with a solution of high concentration. Therefore, dispensing the high concentration DHB solution by the inkjet technique is preferred also in the point of excellent workability. In addition, DHB is particularly effective in analysis using the above-described apparatus such as AXIMA-QIT (made by SHIMADZU CORPORATION). In such an apparatus, it is possible to conduct second or higher of multi-stage MS. This makes it possible to identify a molecule to be measured.

### [6-2. Dispensing pitch]

By two-dimensionally conducting the above dispensing operation over a certain range of the biological sample, it is possible to achieve imaging based on a distribution of a targeted biological molecule on mass, namely mass spectrometric imaging.

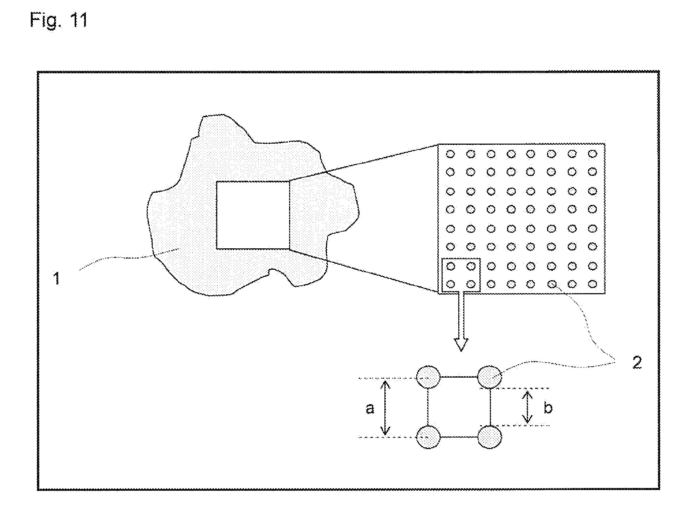

Here, Fig. 11 shows a schematic view of a biological sample 1 and a crystal (or a liquid droplet) of matrix 2. For example, at the same dispensing pitch a, the larger the diameter of a crystal (or a liquid droplet), the narrower the interval b between crystals (or a liquid droplets) becomes. At the same crystal (or a liquid droplets) diameter, the smaller the dispensing pitch a, the narrower the interval b between crystals (or a liquid droplet) is. A plurality of matrix crystals arranged in the vertical and horizontal directions in Fig. 11 are formed on the sample. For achieving this, dispensing may be repeated for every site, or dispensing for plural sites at once by using a means having a plurality of inkjet nozzles.

In the method for an analysis of the present invention, when mass spectrometric imaging is conducted, it is possible to produce crystals in which the matrix is uniformly deposited at any position on the biological sample, by appropriately setting a dispensing pitch. Such a dispensing pitch is 100 to 200 µm, more preferably 125 to 200 µm, and further preferably 150 to 175 µm. In the present invention, the vertical and horizontal pitches in Fig. 11 may be set within such a range. Below the above range, an interval between liquid droplets tends to be too narrow, and the liquid droplets dropped by the dispensing operation tends to associate to become a large Liquid droplet, so that a non-uniform crystal tends to generate. Over the above range, the crystal distribution tends to be too sparse and the area where ionization does not occur by laser irradiation in measurement tends to increase, so that an analytical result of a small information amount tends to be obtained.

By selecting such a dispensing pitch, it is possible to ionize the biological molecule to be measured on the biological sample evenly and effectively, and to find the localization as well as the precise positional information. This will be very useful when the method of the present invention is intended, for example, for an analysis of brain having an especially complicated structure.

When matrix dispensing according to a conventional method is conducted, an operation of visually checking the part where uniform crystals generate with a CCD camera or the like, and irradiating the checked position with a laser beam is often executed. However in the present invention, since high concentration DHB is dispensed at a pitch as described above, it becomes possible to produce crystals in which a matrix is uniformly deposited in any position on the biological sample. This means that ionization occurs uniformly at any position, and is preferred in the point that visual check and restriction of measurement conditions are no longer required.

### EXAMPLES

In the following, the present invention will be explained in detail by way of Examples. However, the present invention will not be limited to these Examples. In the following, an amount represented in % is an amount based on volume unless otherwise specified.

### [Comparative Example 1: Mass spectrometry from paraffin section using conventional method for deparaffinization]

A 10 µm-thick section was prepared from a paraffin block of a mouse brain by means of a microtome, stuck on an indium tin oxide-coated slide glass 8-12ohms (Aldrich), and extended and dried by an extender (50°C, 1 hour). As a conventional deparaffinization treatment, a 100% xylene treatment of 5 minutes was conducted three times. Subsequently, after conducting a 100% ethanol treatment of 5 minutes twice, 90% ethanol treatment of 5 minutes, 80% ethanol treatment of 5 minutes and 70% ethanol treatment of 5 minutes at room temperature as a hydration treatment, the section was dried in a desiccator.

To the obtained deparaffinized section, 100 µg/mL trypsin (in 10 mM NaHCO₃ aqueous solution) was dispensed with the use of CHIP-1000 (SHIMADZU CORPORATION), and allowed to react for 3 hours in an incubator at 37°C. Next, on the section, 50 mg/mL DHB (Wako Pure Chemical Industries, Ltd.) (in 50% acetonitrile - 0.1% trifluoroacetic acid aqueous solution) was dispensed and overlaid at a pitch of 150 µm using a CHIP-1000. Concretely, 5 droplets x 15 cycles for 1 spot, a total of 7500 pL was dispensed and overlaid. As an index, Bradykinin (1 pmol/1.5 mm x 1.5 mm) was contained in the DHB solution. Subsequently, measurement by an AXIMA-QIT (made by SHIMADZU CORPORATION) was conducted under the condition of 100 spots of 450 µm x 450µm at a pitch of 50 µm, 2 profiles for each spot, and a total of 200 profiles. Peak picking of the result was conducted by a Mascot Distiller (Matrix Science, Ltd.)

### [Example 1: Mass spectrometry from paraffin section using method for deparaffinization (heating time: 10 minutes) of the present invention

A 10 µm-thick section was prepared from a paraffin block of a mouse brain by means of a microtome, stuck on an indium tin oxide coated slide glass 8-12ohms (Aldrich), and extended and dried by an extender (50°C, 1 hour). A dying pot accommodating xylene was warmed in advance in a water bath, and a dry paraffin section was put into the dying pot when the temperature of xylene reached a temperature determined as an examination condition, and left still for 10 minutes. As the temperature of xylene, the conditions of 55°C, 60°C, and 65°C were examined. Next, as the hydration treatment, after conducting a 100% ethanol treatment of 5 minutes twice, 90% ethanol treatment of 5 minutes, 80% ethanol treatment of 5 minutes and 70% ethanol treatment of 5 minutes at room temperature, the section was dried in a desiccator.

To the obtained deparaffinized section, 100 µg/mL trypsin (in 10 mM NaHCO₃ aqueous solution) was dispensed with the use of CHIP-1000 (SHIMADZU CORPORATION), and allowed to react for 3 hours in an incubator at 37°C. Next, on the section, 50 mg/mL DHB (Wako Pure Chemical Industries, Ltd.) (in 50% acetonitrile - 0.1% trifluoroacetic acid aqueous solution) was dispensed and overlaid at a pitch of 150 µm using a CHIP-1000. Concretely, 5 droplets x 15 cycles for 1 spot, a total of 7500 pL was dispensed and overlaid. As an index, Bradykinin (1 pool/1.5 mm x 1,5 mm) was contained in the DHB solution. Subsequently, measurement by an AXIMA-QIT (made by SHIMADZU CORPORATION) was conducted under the condition of 100 spots of 450 µm x 450 µm at a pitch of 50 µm, 2 profiles for each spot, and a total of 200 profiles. Peak picking of the result was conducted by a Mascot Distiller (Matrix Science, Ltd.)

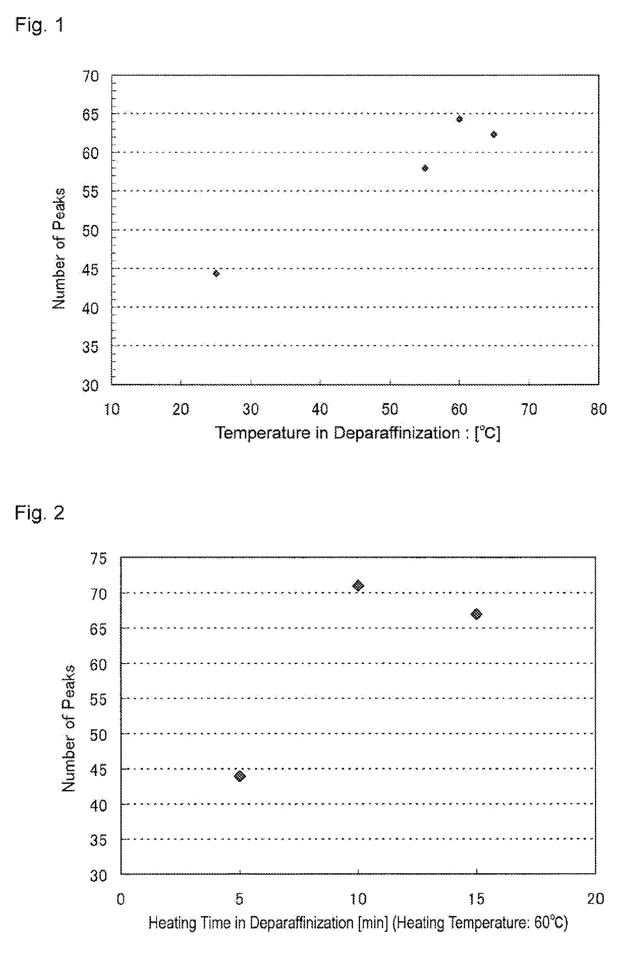

### [Fig. 1: Examination of temperature condition of deparaffinization]

Fig. 1 shows results of the above Comparative Example 1 and Example 1. Concretely, Fig. 1 is a graph showing a result of mass spectrometry from a paraffin section using a conventional method for deparaffinization (Comparative Example 1), and a result of mass spectrometry from a paraffin section using a method for deparaffinization (heating time: 10 min.) according to the present invention (Example 1) in the form of relation between a temperature at the time of deparaffinization and the number of peaks in mass spectrum. In Fig. 1, the horizontal axis represents temperature (°C) at the time of deparaffinization, and the vertical axis represents the number of peaks. These results demonstrate that a larger number of peaks were detected by conducting heating.

### [Example 2: Mass spectrometry from paraffin section using method for deparaffinization (heating temperature: 60°C) of the present invention]

A 10 µm-thick section was prepared from a paraffin block of a mouse brain by means of a microtome, stuck, on an indium tin oxide coated slide glass 8-12ohms (Aldrich), and extended and dried by an extender (50°C, 1 hour). A dying pot accommodating xylene was warmed in advance in a water bath, and a dry paraffin section was put into the dying pot when the temperature of xylene reached 60°C, and left still. As the heating time, the conditions of 5 min, 10 min, and 15 min were examined. Next, as the hydration treatment, after conducting a 100% ethanol treatment of 5 minutes twice, 90% ethanol treatment of 5 minutes, 80% ethanol treatment of 5 minutes and 70% ethanol treatment of 5 minutes at room temperature, the section was dried in a desiccator.

To the obtained deparaffinized section, 100 µg/mL trypsin (in 10 mM NaHCO₃ aqueous solution) was dispensed with the use of CHIP-1000 (SHIMADZU CORPORATION), and allowed to react for 3 hours in an incubator at 37°C_{.} Next, on the section, 50 mg/mL DHB (Wako Pure Chemical Industries, Ltd.) (in 50% acetonitrile - 0.1% trifluoroacetic acid aqueous solution) was dispensed and overlaid at a pitch of 150 µm using a CHIP-1000. Concretely, 5 droplets x 15 cycles for 1 spot, a total of 7500 pL was dispensed and overlaid. As an index, Bradykinin (1 pool/1.5 mm x 1.5 mm) was contained in the DHB solution. Subsequently, measurement by an AXIMA-QIT (made by SHIMADZU CORPORATION) was conducted under the condition of 100 spots of 450 µm x 450 µm at a pitch of 50 µm, 2 profiles for each spot, and a total of 200 profiles. Peak picking of the result was conducted by a Mascot Distiller (Matrix Science, Ltd.)

### [Fig. 2: Examination of time condition of deparaffinization]

Fig. 2 shows a result of the above Example 2. Concretely, Fig. 2 is a graph showing a result of mass spectrometry from a paraffin section using a method for deparaffinization (heating temperature: 60°C) according to the present invention (Example 2) in the form of relation between a heating time at the time of deparaffinization and the number of peaks in mass spectrum. In Fig. 2, the horizontal axis represents heating time (min.) at the time of deparaffinization, and the vertical axis represents the number of peaks.

### [Example 3: Mass spectrometry from paraffin section using method for deparaffinization (heating condition: 60°C-10 min.) of the present invention]

A 10 µm-thick section was prepared from a paraffin block of a mouse brain by means of a microtome, stuck on an indium tin oxide-coated slide glass 8-12ohms (Aldrich), and extended and dried by an extender (50°C, 1 hour). A dying pot accommodating xylene was warmed in advance in a water bath, and a dry paraffin section was put into the dying pot when the temperature of xylene reached 60°C, and left still for 10 minutes. As the hydration treatment, after conducting a 100% ethanol treatment of 5 minutes twice, 90% ethanol treatment of 5 minutes, 80% ethanol treatment of 5 minutes and 70% ethanol treatment of 5 minutes at room temperature, the section was dried in a desiccator.

To the obtained deparaffinized section, 100 µg/mL trypsin (in 10 mM NaHCO₃ aqueous solution) was dispensed with the use of CHIP-1000 (SHIMADZU CORPORATION), and allowed to react for 3 hours in an incubator at 37°C. Next, on the section, 50 mg/mL DHB (Wako Pure Chemical Industries, Ltd.) (in 50% acetonitrile - 0.1% trifluoroacetic acid aqueous solution) was dispensed and overlaid at a pitch determined as an examination condition using a CHIP-1000. Concretely, 5 droplets x 15 cycles for 1 spot, a total of 7500 pL was dispensed and overlaid. As the dispensing pitch, the conditions of 200 µm, 175 µm, 150 µm, 125 µm and 100 µm were examined. As an index, Bradykinin (1 pmol/1.5 mm x 1.5 nm) was contained in the DHB solution. Subsequently, measurement by an AXIMA-QIT (made by SHIMADZU CORPORATION) was conducted under the condition of 100 spots of 450 µm x 450µm at a pitch of 50 µm, 2 profiles for each spot, and a total of 200 profiles. Peak picking of the result was conducted by a Mascot Distiller (Matrix Science, Ltd.)

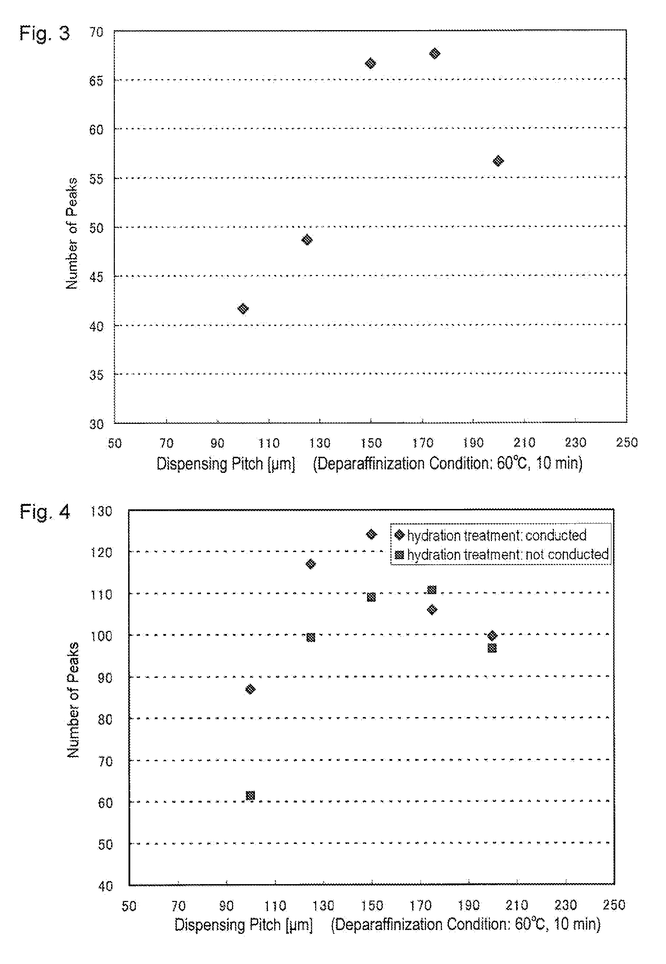

### [Fig. 3: Examination of dispensing pitch]

Fig. 3 shows a result of the above Example 3. Concretely, Fig. 3 is a graph showing a result of mass spectrometry from a paraffin section using a method for deparaffinization (heating condition: 60°C-10 min.) according to the present invention (Example 3), in the form of relation between a dispensing pitch of a matrix solution and the number of peaks in mass spectrum. In Fig. 3, the horizontal axis represents a dispensing pitch (µm) and the vertical axis represents the number of peaks.

### [Example 4: Mass spectrometry from paraffin section using inventive method for analysis (deparaffinization: 60°C-10 min.) not executing hydration treatment]

A 10 µm-thick section was prepared from a paraffin block of a mouse brain by means of a microtome, stuck on an indium tin oxide coated slide glass 8-12ohms (Aldrich), and extended and dried by an extended (50°C, 1 hour). A dying pot accommodating xylene was warmed in advance in a water bath, and a dry paraffin section was put into the dying pot when the temperature of xylene reached 60°C, and left still for 10 minutes. The paraffin section was put out from the post, and xylene was vaporized.

To the obtained deparaffinized section, 100 µg/mL trypsin (in 10 mM NaHCO₃ aqueous solution) was dispensed with the use of CHIP-1000 (SHIMADZU CORPORATION), and allowed to react for 3 hours in an incubator at 37°C. Next, on the section, 50 mg/mL DHB (Wako Pure Chemical Industries, Ltd.) (in 50% acetonitrile - 0.1% trifluoroacetic acid aqueous solution) was dispensed and overlaid at a pitch determined as an examination condition using a CHIP-1000. Concretely, 5 droplets x 15 cycles for 1 spot, a total of 7500 pL was dispensed and overlaid. As the dispensing pitch, the conditions of 200 µm, 175 µm, 150 µm, 125 µm and 100 µm were examined. As an index, Bradykinin (1 pmol/1.5 mm x 1.5 mm) was contained in the DHB solution. Subsequently, measurement by an AXIMA-QIT (made by SHIMADZU CORPORATION) was conducted under the condition of 100 spots of 450 µm x 450 µm at a pitch of 50 µm, 2 profiles for each spot, and a total of 200 profiles. Peak picking of the result was conducted by a Mascot Distiller (Matrix Science, Ltd.)

### [Fig. 4: Comparison and examination of presence/absence of hydration treatment]

Fig. 4 shows a result of above Example 4, together with a result corresponding to Example 3 (namely, the result obtained by separately conducting the same operation as Example 3). Fig. 4 is a graph showing a result of spectrometry, from a paraffin section according to a method for an analysis of the present invention in which a hydration treatment is conducted (deparaffinization condition: 60°C-10 min.) (Example 3) and a result of mass spectrometry from a paraffin section according to a method for an analysis of the present invention in which a hydration treatment is not conducted (deparaffinization condition: 60°C-10 min.) (Example 4), in the form of relation between a dispensing pitch of a matrix solution and the number of peaks in mass spectrum. In Fig. 4, the horizontal axis represents a dispensing pitch (µm), and the vertical axis represents the number of peaks.

### [Comparative Example 2: Mass spectrometry from frozen section according to conventional method for an analysis]

A 10 µm-thick section was prepared from a frozen block of a mouse brain by means of a cryostat, stuck on an indium tin oxide coated slide glass 8-12ohms (Aldrich), and dried in air. After a treatment with 70% ethanol for 5 minutes at room temperature, the section was dried in a desiccator.

To the frozen section, 100 µg/mL, trypsin (in 10 mM NaHCO₃ aqueous solution) was dispensed with the use of CHIP-1000 (SHIMADZU CORPORATION), and allowed to react for 3 hours in an incubator at 37°C. Next, on the section, 50 mg/mL DHB (Wako Pure Chemical Industries, Ltd.) (in 50% acetonitrile - 0.1% trifluoroacetic acid aqueous solution) was dispensed and overlaid at a pitch of 150 µm using a CHIP-1000. Concretely, 5 droplets x 15 cycles for 1 spot, a total of 7500 pL was dispensed and overlaid. As an index, Bradykinin (1 pmol/1.5 mm x 1.5 mm) was contained in the DHB solution. Subsequently, measurement by an AXIMA-QIT (made by SHIMADZU CORPORATION) was conducted under the condition of 100 spots of 450 µm x 450 µm at a pitch of 50 µm, 2 profiles for each spot, and a total of 200 profiles. Peak picking of the result was conducted by a Mascot Distiller (Matrix Science, Ltd.)

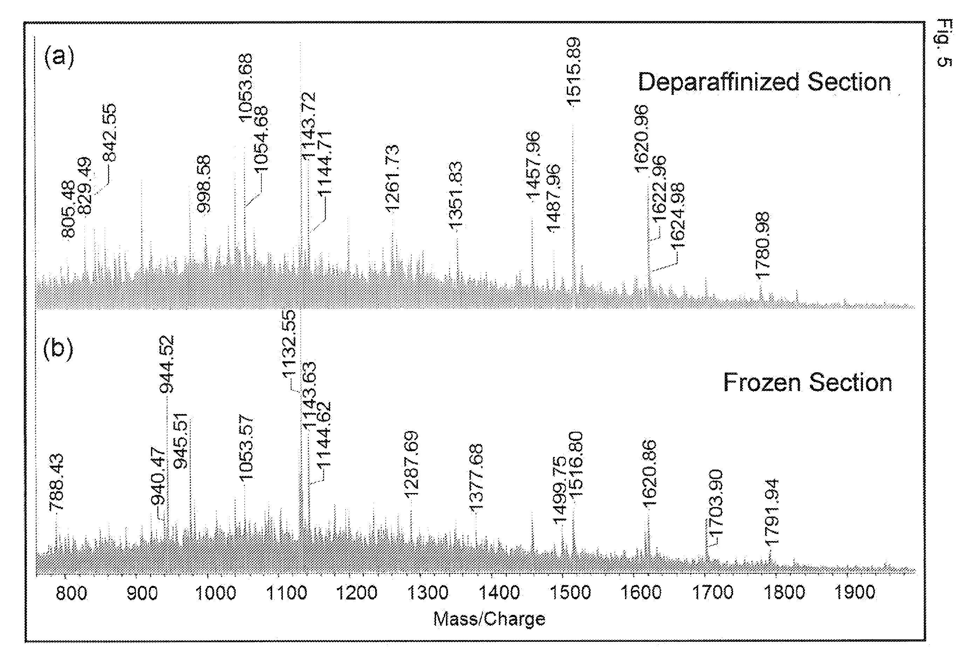

### [Fig. 5: Comparison and examination of mass spectrums obtained from frozen section and from paraffin section]

Fig. 5(a) is a mass spectrum obtained as a result of mass spectrometry from a paraffin section according to a method for an analysis of the present invention (Example 3: deparaffinization condition is 60°C, 10 min.), and Fig. 5(b) is a mass spectrum obtained as a result of mass spectrometry from a frozen section according to a conventional method for an analysis (Comparative Example 2). In Fig. 5(a) and Fig. 5(b), the horizontal axis represents Mass/Charge, and the vertical axis represents ionic intensity. As shown in Fig. 5, according to the method of the present invention, even in the case of a sample prepared from a paraffin section, a mass spectrum of the same quality or even better quality in an information amount compared to a sample from a frozen section can be obtained.

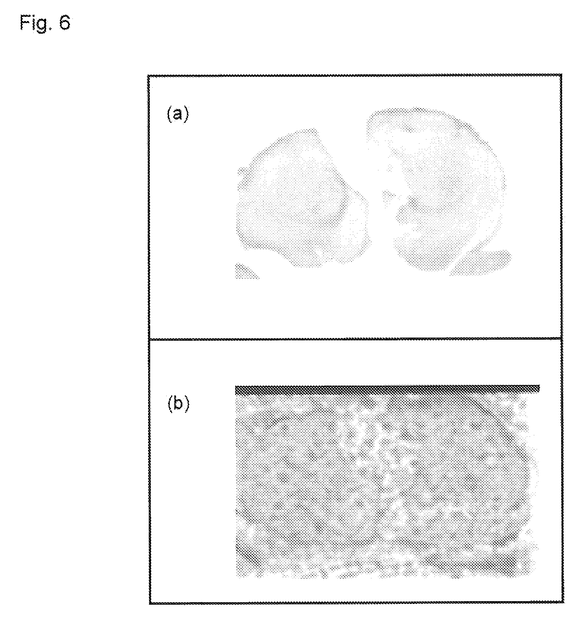

### [Figs. 6 to 8: Mass spectrometric imaging]

Fig. 6(a) shows an actual image of a paraffin section analyzed in the present invention, and Fig. 6(b) shows an actual image of a paraffin section after dispensing of a matrix.

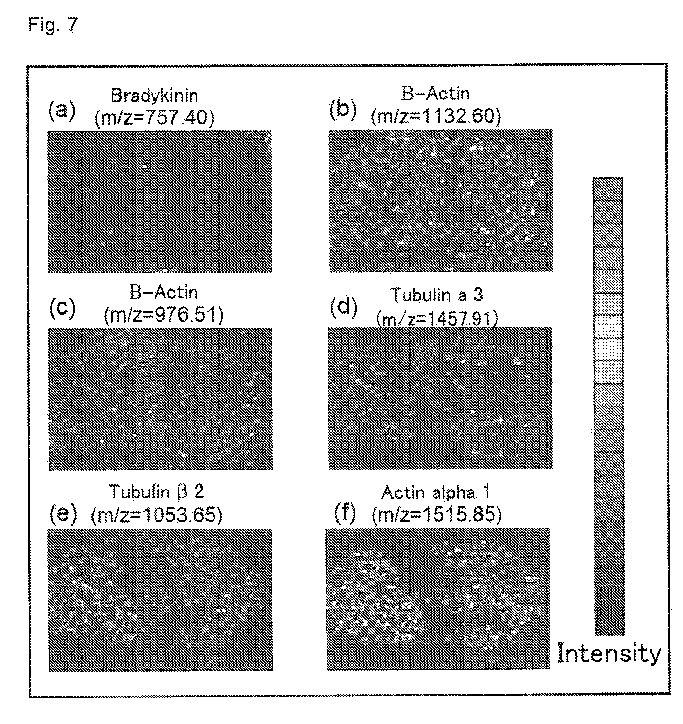

Fig. 7 shows images (a) to (f) obtained by mass spectrometric imaging of the paraffin section according to a method for an analysis of the present invention of Example 3 (deparaffinization condition: 60°C-10 min., hydration treatment: conducted, matrix solution dispensing pitch: 150 µm). In Fig. 7, (a) represents Bradykinin which is an index, (b) represents a component having m/z=1132.60 of β-Action, (c) represents a component having m/z=976.51 of β-Action, (d) represents a component having m/z=1457.91 of Tubulin a3, (e) represents a component having m/z=1053.65, and (f) represents a component having m/z=1515.85, with different colors for different peak intensities of the mass spectrum.

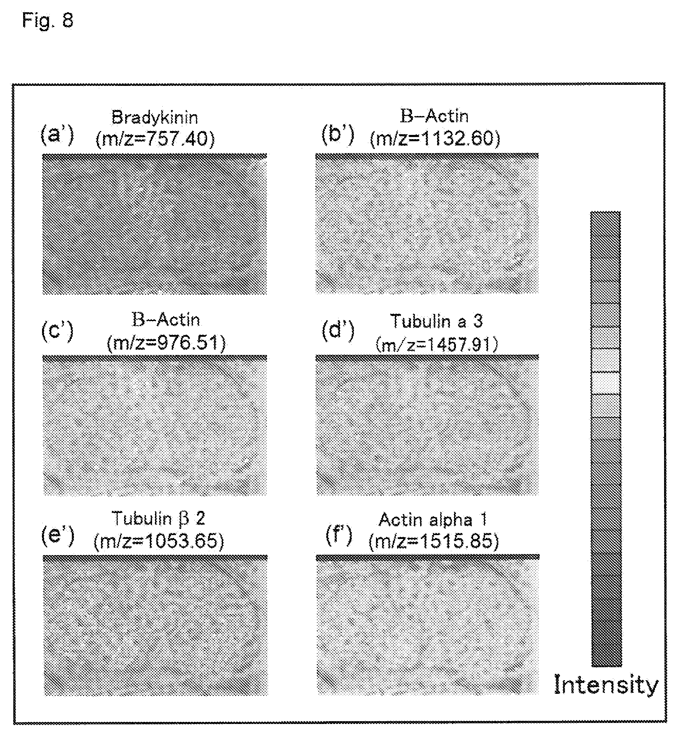

Fig. 8 shows images (a') to (f') obtained by superimposing an actual image (b) after dispensing of matrix in Fig. 6, on the images (a) to (f) of Fig. 7.

### [Reference example 1: Preparation of matrix crystal in deparaffinized section]

A matrix crystal was prepared on a section obtained by subjecting a mouse brain paraffin section to deparaffinization by the deparaffinization method according to Example 3, in the following manner.

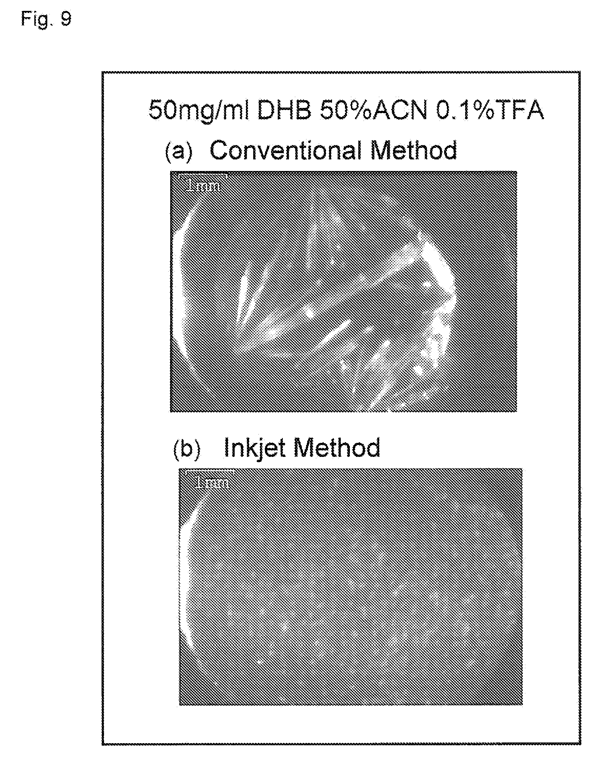

A 50 mg/mL of 2,5-dihydroxybenzoic acid solution was prepared using a 50% acet-onitrile-0.1% trifluoroacetic acid aqueous solution as a solvent. The prepared high concentration matrix solution was added dropwise on a deparaffinized section using a micro pipette. A photograph of the resultant crystal is shown in Fig. 9(a). Further, the prepared high concentration matrix solution was added dropwise on a deparaffinized section using a chemical printer CHIP-1000 (SHIMADZU CORPORATION). A photograph of the resultant crystal is shown in Fig. 9(b).

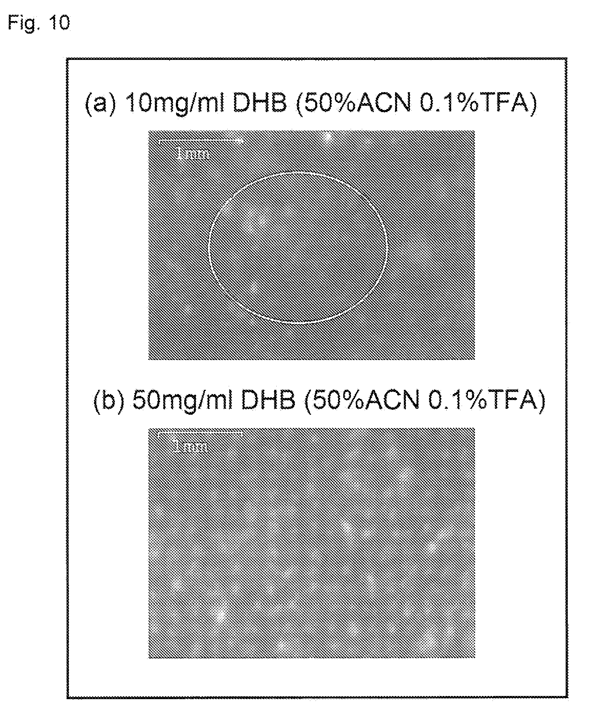

10 mg/mL of a 2,5-dihydroxybenzoic acid solution was prepared using a 50% acetonitrile-0.1% trifluoroacetic acid aqueous solution as a solvent. The prepared low concentration matrix solution was added dropwise on a deparaffinized section using a chemical printer CHIP-1000. A photograph of the resultant crystal is shown in Fig. 10(a). Taking a notice of the part surrounded by the circle, for example, it can be found that the crystals generate unevenly.

Fig. 10 (b) is a crystal obtained by dropping the above 50 mg/mL high concentration matrix solution on a deparaffinized section using a chemical printer CHIP-1000, and is a part of the above Fig. 9(b) enlarged to the same scale as Fig. 10(a).

These photographs demonstrate that by dropping the high concentration matrix solution by an inkjet mechanism, uniform crystals generate evenly.

While concrete forms within the scope of the present invention have been described in the above Examples, the present invention may be practiced in various other forms without limited to these. Accordingly, the above Examples are given merely for exemplification in every terms, and should not be interpreted in a limitative manner. Further, any modifications within equivalents of claims are included in the scope of the present invention.

## Claims

1. A method for deparaffinization of paraffin-embedded specimen comprising the steps of:
exposing a biological sample by subjecting a specimen in which the biological sample is embedded in paraffin, to an organic solvent that is compatible with the paraffin under heating condition, to make the paraffin be melted and dissolved in the organic solvent; and
removing the paraffin from the specimen by separating the organic solvent dissolving the paraffin, from the exposed biological sample.

2. The method for deparaffinization of a paraffin-embedded specimen according to claim 1, wherein in the step of exposing the biological sample, the specimen in which the biological sample is embedded in the paraffin is immersed and retained in the organic solvent that is heated, and
in the step of removing the paraffin, the specimen retained in the organic solvent is drawn up from the organic solvent.

3. The method for deparaffinization of a paraffin-embedded specimen according to claim 1, wherein the heating condition is a temperature condition of 45 to 70°C.

4. The method for deparaffinization of a paraffin-embedded specimen according to claim 1, wherein the organic solvent is selected from the group consisting of xylene, chloroform, diethyl ether, lemosol, and alcohols.

5. The method for deparaffinization of a paraffin-embedded specimen according to claim 1, wherein the specimen is held on a surface of an electrically conductive support.

6. The method for deparaffinization of a paraffin-embedded specimen according to claim 1, wherein the specimen is derived from a living body suffering from disease selected from the group consisting of cancer, Alzheimer disease, Parkinson disease, ischemic cerebrovascular disease, and ischemic cerebrovascular disease.

7. The method for deparaffinization of a paraffin-embedded specimen according to claim 1, wherein the specimen is a specimen for analyzing pharmacokinetics.

8. A method for analysis of a paraffin-embedded specimen comprising the steps of:
removing a paraffin from a specimen in which a biological sample is embedded in the paraffin using the method according to claim 1; and
measuring the biological sample by a mass spectrometer.

9. The method for analysis of a paraffin-embedded specimen according to claim 8, further comprising the step of subjecting the biological sample to a digestion treatment, after the step of removing paraffin and before the step of measuring by a mass spectrometer.

10. The method for analysis of a paraffin-embedded specimen according to claim 8, further comprising the step of subjecting the biological sample to a hydration treatment, after the step of removing paraffin and before the step of measuring by a mass spectrometer.

11. A method for analysis of a paraffin-embedded specimen according to claim 8, further comprising a step of subjecting the biological sample to a hydration treatment and a digestion treatment, after the step of removing paraffin and before the step of measuring by a mass spectrometer.

12. The method for analysis of a paraffin-embedded specimen according to claim 8, wherein in the step of measuring by a mass spectrometer, a matrix-assisted laser desorption/ionization mass spectrometer is used as the mass spectrometer.

13. The method for analysis of a paraffin-embedded specimen according to claim 12, wherein 2,5-dihydroxybenzoic acid is used as a matrix, a solution of 40 mg/mL to a saturated concentration of the matrix is added dropwise to the biological sample by using an inkjet mechanism, and measurement is conducted using the matrix-assisted laser desorption/ionization mass spectrometer.

14. The method for analysis of a paraffin-embedded specimen according to claim 13, wherein the matrix solution is added dropwise at a pitch of 100 to 200 µm.
